# EUROPEAN PATENT APPLICATION

(11) **EP 4 053 282 A1**
(43) Date of publication of application: **07.09.2022**
(21) Application number: 20882284.1
(22) Date of filing: 26.06.2020
(51) Int. Cl.: C12N 15/70, C12P 13/22

(54) **STRAIN HAVING IMPROVED AROMATIC AMINO ACID PRODUCTION CAPABILITY DUE TO YEEO GENE INACTIVITY**

(30) Priority: 31.10.2019 KR 20190138232
(71) Applicant: Daesang Corporation, Seoul 02586 (KR)
(72) Inventor: SHIN, Won Joo, Icheon-si Gyeonggi-do 17384 (KR); JO, Young Il, Seoul 04995 (KR); LEE, Sun Hee, Yongin-si Gyeonggi-do 16923 (KR); KIM, Hyun Young, Yongin-si Gyeonggi-do 17095 (KR); KIM, Yong Soo, Suwon-si Gyeonggi-do 16694 (KR); YANG, Cheol Min, Seoul 07958 (KR)
(74) Representative: Nony
(86) International application number: PCT/KR2020/008405
(87) International publication number: WO 2021/085794

(57) **Abstract**

Disclosed is a mutant strain having improved aromatic amino acid production capability due to inactivation or weakening of activity of an FMN/FAD exporter protein which is expressed by yeeO gene.

## Description

### Technical Field

The present invention relates to a strain having improved aromatic amino acid production capability due to inactivation of yeeO gene.

### Background Art

Aromatic amino acids, particularly L-tryptophan and L-phenylalanine, are important amino acids for feed and are high value-added industries having an annual worldwide market size of 300 billion dollars.

Aromatic amino acids are produced using recombinant strains, and studies have been actively conducted to increase their production. Chorismate is a precursor required in the aromatic amino acid biosynthesis pathways, and in order to produce chorismate, phosphoenolpyruvate (PEP), erythrose-4-phosphate (E4P), the sub-substrate phosphoribosyl pyrophosphate (PRPP), serine, glutamine and the like are needed. Thus, in a conventional art, studies on enhancement of the biosynthetic pathway of E4P, PEP or PRPP have been conducted to improve L-tryptophan production.

However, it has not been reported that the production of aromatic amino acids by a strain is increased by inhibiting the export of flavin mononucleotide (FMN) and flavin adenine dinucleotide (FAD) in the strain.

### [Prior Art Documents]

### [Patent Documents]

(Patent Document 0001) Korean Patent No. 10-1830002 (February 9, 2018)

### DISCLOSURE

### Technical Problem

According to one embodiment, there is provided a strain having improved aromatic amino acid production capability as a result of inhibiting the expression of the yeeO gene.

### Technical Solution

One aspect provides a mutant strain having improved aromatic amino acid production capability due to inactivation or weakening of activity of the FMN/FAD exporter protein which is expressed by the yeeO (FMN/FAD exporter) gene.

The yeeO gene may consist of the nucleotide sequence of SEQ ID NO: 1.

The yeeO protein is an exporter that exports flavin mononucleotide (FMN) and flavin adenine dinucleotide (FAD). The relationship of the yeeO gene with an aromatic amino acid production pathway such as the tryptophan pathway, or the effect of the yeeO gene on the aromatic amino acid production pathway is unknown. Nevertheless, the present inventors have found that, when the export of FMN and FAD is inhibited by inhibiting the expression of the yeeO protein in a strain, the proportion of D-ribulose 5-phosphate used for FMN and FAD production may decrease, and D-ribulose-5-phosphate may be concentrated on enhancing the erythrose-4-phosphate (E4P) pool, thereby improving the aromatic amino acid productivity of the strain.

As used herein, the term "weakening of activity" means that the expression level of a gene of interest is decreased compared to the original expression level thereof. This weakening of activity includes: a case in which the activity of an enzyme itself is decreased compared to the activity of the enzyme, originally possessed by the microorganism, through substitution, insertion or deletion of one or more nucleotides in the nucleotide sequence of the gene encoding the enzyme, or a combination thereof; a case in which the overall activity of the enzyme in the cell is lower than that in a native strain or a strain before modification due to inhibition of expression or translation of the gene encoding the enzyme; and a combination thereof.

As used herein, the term "inactivation" refers to a case in which a gene encoding a protein such as an enzyme is not expressed at all compared to that in a native strain or a strain before modification, and has no activity even if it is expressed.

As used herein, the term "increased expression" means that the expression level of a gene of interest is increased compared to the original expression level of the gene. If a gene whose expression is to be increased is not present in a strain before mutation, the expression may be increased by introducing one or more genes into the chromosome of the strain, and if a gene whose expression is to be increased is present in a strain before mutation, one or more genes may be additionally introduced into the strain, or the strain may be genetically engineered to increase the expression level of the existing gene.

In the present invention, a method of modifying an expression regulatory sequence may be performed by inducing a modification in the expression regulatory sequence by deletion, insertion, or non-conservative or conservative substitution of one or more nucleotides in the nucleic acid sequence of the expression regulatory sequence, or a combination thereof, or may be performed by substituting the sequence with a weaker promoter. Examples of the expression regulatory sequence include a promoter, an operator sequence, a sequence encoding a ribosome-binding site, and a sequence for regulating the termination of transcription and translation.

In addition, a method of modifying a gene sequence on the chromosome may be performed by inducing a modification in the sequence by deletion, insertion, non-conservative or conservative substitution, or a combination thereof in the gene sequence so as to further weaken the enzyme activity, or may be performed by replacing the sequence with a gene sequence improved to have weaker activity or with a gene sequence improved to have no activity.

According to one embodiment, the aromatic amino acid may be at least one of L-tyrosine, L-tryptophan, and L-phenylalanine.

According to one embodiment, the mutant strain may be obtained by insertion, substitution or deletion of one or more nucleotides in the nucleotide sequence of the yeeO gene.

According to one embodiment, the mutant strain may bederived from a strain of the genus Escherichia.

According to one embodiment, the strain of the genus Escherichia may be *Escherichia coli,* for example, a strain deposited under accession number KFCC11660P or KCCM10016.

Another aspect provides a method for producing an aromatic amino acid, comprising steps of: culturing the mutant strain in a medium; and recovering an aromatic amino acid from the cultured strain and the medium.

The strain according to the present invention may be cultured through a culture method known in the art. As the medium, a natural medium or a synthetic medium may be used. Examples of the carbon source of the medium include glucose, sucrose, dextrin, glycerol, starch, etc., and examples of the nitrogen source of the medium include peptone, meat extract, yeast extract, dried yeast, soybean cake, urea, thiourea, ammonium salt, nitrate and other organic or inorganic nitrogen-containing compounds, but the carbon and nitrogen sources are not limited to these components.

As inorganic salts contained in the medium, phosphates, nitrates, carbonates, chlorides, etc. of magnesium, manganese, potassium, calcium, iron, etc. may be used, without being limited thereto. In addition to the components of the carbon source, nitrogen source and inorganic salt, amino acids, vitamins, nucleic acids and related compounds may be added to the medium.

The temperature of the culture medium may be usually 27 to 40°C, more preferably 30 to 37°C, without being limited thereto. The culture may be continued until the useful substances are produced in desired amounts. The culture time may preferably be 10 to 100 hours, without being limited thereto.

In the step of recovering the aromatic amino acid, the desired amino acid may be recovered from the culture medium using a suitable method known in the art, depending on the method used for culture of the microorganism of the present invention, for example, a batch, continuous or fed-batch culture method. The step of recovering may include a purification process.

According to one embodiment, the aromatic amino acid may be at least one of L-tryptophan and L-phenylalanine.

### Advantageous Effects

According to one embodiment, it is possible to increase the production of aromatic amino acids by inactivation or weakening of activity of the FMN/FAD exporter protein which is expressed by the yeeO gene of the strain.

### Mode for Invention

Hereinafter, one or more specific embodiments will be described in more detail with reference to examples. However, these examples are for illustrating one or more embodiments, and the scope of the present invention is not limited to these examples.

### Example 1: Construction of yeeO gene-deleted strains

yeeO gene-inactivated mutant strains were constructed from parent strains (accession numbers: KFCC11660P and KCCM10016) by a one-step inactivation method (One-step inactivation of chromosomal genes in Escherichia coli K-12 using PCR products, Datsenko KA, Wanner BL., Proc Natl Acad Sci USA. 2000 Jun 6;97(12):6640-5).

The KFCC11660P strain and the KCCM10016 strain are *Escherichia coli* strains. For homologous recombination of the fourth fragment, pKD46 (GenBank accession number AY048746), a Red recombinase plasmid, was introduced into each of the strains, and pKD46 was removed before introduction of pCP20.

The yeeO gene was deleted by homologous recombination between the yeeO gene and a DNA fragment containing an antibiotic resistance gene, and then the yeeO gene was inactivated by removing the antibiotic resistance gene from the recombined DNA fragment. The specific process is as follows.

### (1) Construction of first fragment

PCR reaction (total volume: 50 µl) was performed using a pKD13 plasmid (Genbank accession number: AY048744) and a primer pair of yeeO_PF and yeeO_PR having a portion of the yeeO gene sequence shown in Table 1 below and a portion of the pKD13 plasmid sequence under the following conditions, thus obtaining a first amplified fragment of about 1.4 kb in length: one cycle of 5 min at 95°C, and then 30 cycles, each consisting of 30 sec at 95°C, 30 sec at 58°C, and 2 min at 72°C, followed by 5 min at 72°C and 10 min at 12°C. The first fragment contained the kanamycin resistance gene derived from the pKD13 plasmid.

**[Table 1]**

| | SEQ ID NO | Sequence |
|---|---|---|
| yeeO | 1 | ttgttgaggc acatcttaac ggcgaaaaat cttttgtcaa acccgatttt taaattccccaactgtttgc cgtttctatc aacagtttgt tgcatttgca gacaatttgt tggcgaaaat ctttgcagct ttgctgattc tccctcatta tttgaaatgt ggtttcactt tctgcaatta aggtcggctt tgaatatctc ctctgcttta cgccaggttg ttcacggcac tcgctggcac gctaaacgca agagctacaa agtgttgttc tggcgcgaga taaccccgct tgctgttcct atcttcatgg agaatgcctg tgtcctgttg atgggggttc tgagcacttt tctggtcagc tggctgggaa aagatgcgat ggccggcgtg ggattggcgg acagcttcaa tatggtcatt atggcttttt ttgctgctat cgatcttggt actactgtcg ttgtggcatt tagtctcggt aagcgggatc gacgacgagc gagggtggcg acgcggcagt cattggtgat catgacgttg tttgccgtac tgttggcaac gcttattcat cattttggcg aacaaattat tgatttcgtc gcgggtgatg ccacgacaga agttaaagca ctggcgttga cttatctgga gctgacggta |
| | | ctcagttatc cagcagctgc catcactctt attggtagcg gggcacttcg tggtgcaggg aatacgaaaa taccgctatt gattaacggt agcctgaata ttcttaatat tattattagc ggcatattga tttacggcct tttctcctgg ccgggactgg gatttgtcgg ggcagggctg ggtttaacca tttctcgtta tattggcgca gttgcaattt tgtgggtgct ggcgattggt tttaatcctg cgctaaggat ttcgttaaag agctatttta aaccgctgaa ttttagcatt atctgggaag tcatggggat tggtattccc gcgagtgtcg aatcagtgtt atttaccagt ggtcggttat taacccaaat gttcgttgcc gggatgggga ccagtgttat tgccggaaat tttatcgcgt tttcaattgc ggctcttatc aacttacccg gaagtgcgct cggctctgct tctacgatca ttacaggccg aaggttgggg gtagggcaga tagcgcaagc agagattcag ttgcggcatg tgttctggct ttccactctt ggattaacgg ccatcgcctg gctaacggct ccctttgccg gggttatggc atcgttttac acccaggatc cacaggttaa acatgtcgtt gtgattctga tttggctaaa tgctttattt atgcctattt ggtccgcctc atgggtgcta cccgctggat ttaaaggtgc tcgtgatgcc cgttacgcca tgtgggtttc gatgttgagc atgtggggtt gtcgggttgt agtcggttat gtgctgggaa tcatgcttgg ctggggtgtg gttggtgtct ggatgggaat gtttgccgac tgggctgtgc gggccgtgct gttttactgg cgaatggtta ctggacgttg gctatggaaa taccctcgac ccgagccgca aaagtgtgaa aaaaagccag ttgtgtcgga ataa |
| yeeO_H F1 | 2 | cgtaactatg tctggcgctt |
| yeeO_H R1 | 3 | aaacggcaaa cagttgggga |
| yeeO_P F | 4 | tccccaactg tttgccgttt gtgtaggctg gagctgcttc |
| yeeO_P R | 5 | gtttattccg acacaactgg ctgtcaaaca tgagaattaa |
| yeeO_H F2 | 6 | ccagttgtgt cggaataaac g |
| yeeO_H R2 | 7 | agactcgatt aagcgcacga |
| yeeO_C F | 8 | cctatgttgc tcttgggctt |
| yeeO_C R | 9 | aggcaaagtg gcaattacgc |

### (2) Construction of second fragment

To obtain an upstream fragment of the yeeO gene, PCR reaction (total volume: 50 µl) was performed using the genomic DNA of *E*. *coli* MG1655 as a template and the primers yeeO_HF1 and yeeO_HR1 shown in Table 1 above under the following conditions, thus obtaining a second amplified fragment of about 0.3 kb in length: one cycle of 5 min at 95°C, and then 30 cycles, each consisting of 30 sec at 95°C, 30 sec at 58°C, and 30 sec at 72°C, followed by 5 min at 72°C and 10 min at 12°C.

### (3) Construction of third fragment

To obtain a downstream fragment of the yeeO gene, PCR reaction (total volume: 50 µl) was performed using the genomic DNA of *E*. *coli* MG1655 as a template and the primers yeeO_HF2 and yeeO_HR2 shown in Table 1 above under the following conditions, thus obtaining a third amplified fragment of about 0.3 kb in length: one cycle of 5 min at 95°C, and then 30 cycles, each consisting of 30 sec at 95°C, 30 sec at 58°C, and 30 sec at 72°C, followed by min at 72°C and 10 min at 12°C.

### (4) Construction of fourth fragment

The first fragment, second fragment and third fragment amplified in the above experiment could be ligated into a single fragment due to the complementary sequences of the primers during amplification. These fragments were subjected to PCR (total volume: 50 µl) without primers under the following conditions, thus obtaining a fourth amplified single fragment having a size of about 2 kb: one cycle of 5 min at 95°C, and then 30 cycles, each consisting of 30 sec at 95°C, 30 sec at 58°C, and 2 min and 30 sec at 72°C, followed by 5 min at 72°C and 10 min at 12°C. The fourth fragment contained a portion of the yeeO gene and the kanamycin antibiotic resistance gene. Specifically, it consisted of a portion of the 5' fragment of the yeeO gene, the kanamycin antibiotic resistance gene, and a portion of the 3' fragment of the yeeO gene.

### (5) Introduction of fourth fragment and deletion of yeeO

The obtained fourth fragment was introduced by electroporation into each of the KFCC11660P and KCCM10016 strains, which are *Escherichia* colistrains containing the Red recombinase plasmid pKD46 (GenBank accession number: AY048746). The fourth fragment was replaced with yeeO by homologous recombination using the Lambda Red recombination system, whereby yeeO was deleted.

Thereafter, PCR reaction was performed on the cell line showing kanamycin resistance to confirm whether the yeeO gene was deleted. The PCR reaction (total volume: 20 µl) was performed using the yeeO_CF and yeeO_CR primers shown in Table 1 above under the following conditions: one cycle of 5 min at 95°C, and then 30 cycles, each consisting of 30 sec at 95°C, 30 sec at 55°C, and 3 min at 72°C, followed by 5 min at 72°C and 10 min at 12°C. It was confirmed that, when the original yeeO gene was present, about 2.5 kb (before deletion) was produced, whereas when the fragment was inserted into the chromosome, about 2.2 kb which is a decreased length was produced.

### (6) Antibiotic resistance gene removal and selection

To remove the antibiotic resistance marker gene from the strain in which deletion of the yeeO gene was confirmed, FLP recombination was induced by introducing a pCP20 plasmid into the strain. Thereafter, the yeeO-deleted strain was cultured in LB plate medium with or without antibiotics to confirm that the antibiotic resistance marker gene was removed.

### Example 2: Culture of yeeO-deleted strain and evaluation of aromatic amino acid production

Each of the *E*. *coli* strain KFCC11660PΔyeeO obtained by the method of Example 1 and KFCC11660P was cultured in the tryptophan-producing medium shown in Table 2 below.

In addition, each of the *E*. *coli* strain KCCM10016PΔyeeO obtained by the method of Example 1 and KCCM10016 was cultured in the phenylalanine-producing medium shown in Table 2 below.

For culture, 1 vol% of each of the KFCC11660PΔyeeO, KFCC11660P, KCCM10016ΔyeeO, and KCCM10016 strains was inoculated into a flask containing 10 mL of the tryptophan-producing medium or phenylalanine-producing medium having the composition shown in Table 2 below, and cultured with shaking at 200 rpm at 37°C for 70 hours. Then, the concentrations of L-amino acids obtained from the strains were compared.

**[Table 2]**

| Tryptophan-producing medium | | Phenylalanine-producing medium | |
|---|---|---|---|
| Component | Content | Component | Content |
| Glucose | 80.0 g/L | Glucose | 80.0 g/L |
| (NH₄)₂SO₄ | 20.0 g/L | (NH₄)₂SO₄ | 20.0 g/L |
| K₂HPO₄ | 0.8 g/L | K₂HPO₄ | 1.0g/L |
| K₂SO₄ | 0.4 g/L | KH₂PO₄ | 1.0 g/L |
| MgCl₂ | 0.8 g/L | K₂SO₄ | 0.4 g/L |
| Fumaric acid | 1.0 g/L | MgCl₂ | 1.0 g/L |
| Yeast extract | 1.0 g/L | Fumaric acid | 0.5 g/L |
| (NH₄)₆Mo₇O₂₄ | 0.12 ppm | Yeast extract | 1.0 g/L |
| H₃BO₃ | 0.01 ppm | Glutamic acid | 0.5g/L |
| CuSO₄ | 0.01 ppm | CaCl₂ | 5.00 ppm |
| MnCl₂ | 2.00 ppm | MnCl₂ | 2.00 ppm |
| ZnSO₄ | 0.01 ppm | ZnSO₄ | 1.00 ppm |
| CoCl₂ | 0.10 ppm | CoCl₂ | 0.10 ppm |
| FeCl₂ | 10.00 ppm | FeCl₂ | 10.00 ppm |
| Thiamine_HCl | 20.00 ppm | Thiamine_HCl | 20.00 ppm |
| L-Tyrosine | 200.00 ppm | L-Tyrosine | 200.00 ppm |
| L-phenylalanine | 300.00 ppm | CaCO₃ | 3% |
| CaCO₃ | 3% | - | - |

As a result of the above experiment, as shown in Tables 3 and 4 below, it was confirmed that the production of tryptophan and phenylalanine increased in the case of the strains in which the yeeO gene was inactivated.

Referring to Tables 3 and 4 below, it was confirmed that, when the yeeO gene in the KFCC11660P strain was inactivated, the production of L-tryptophan increased by 10% or more, and when the yeeO gene in the KCCM10016 strain was inactivated, the production of L-phenylalanine increased by 15% or more.

**[Table 3]**

| Strain | L-tryptophan (g/L) |
|---|---|
| KFCC11660P | 4.18 |
| KFCC11660PΔyeeO | 4.65 |

**[Table 4]**

| Strain | L-phenylalanine (g/L) |
|---|---|
| KCCM10016 | 3.48 |
| KCCM10016ΔyeeO | 4.14 |

## Claims

1. A mutant strain having improved aromatic amino acid production capability due to inactivation or weakening of activity of an FMN/FAD exporter protein which is expressed by yeeO gene.

2. The mutant strain of claim 1, wherein the yeeO gene consists of the nucleotide sequence of SEQ ID NO: 1.

3. The mutant strain of claim 1, wherein the aromatic amino acid is at least one of L-tryptophan and L-phenylalanine.

4. The mutant strain of claim 1, which is obtained by insertion, substitution or deletion of one or more nucleotides in the nucleotide sequence of the yeeO gene.

5. The mutant strain of claim 1, which is derived from a strain of the genus Escherichia.

6. The mutant strain of claim 5, wherein the strain of the genus Escherichia is *Escherichia coli.*

7. A method for producing an aromatic amino acid, comprising steps of:
culturing the mutant strain of claim 1 in a medium; and
recovering an aromatic amino acid from the cultured mutant strain and the medium.

8. The method of claim 7, wherein the aromatic amino acid is at least one of L-tryptophan and L-phenylalanine.


<110>     Daesang Corporation

<120>     Strain with Improved Aromatic Amino Acid Production Capacity by
          yeeO Gene Inactivation

<130>     PX200023PCT

<150>     KR 10-2019-0138232
<151>     2019-10-31

<160>     9

<170>     KoPatentIn 3.0

<210>     1
<211>     1644
<212>     DNA
<213>     Artificial Sequence

<220>
<223>     yeeO "YEEO-MONOMER" (complement(2058203..2059846)) Escherichia
          coli K-12 substr. MG1655

<400>     1
ttgttgaggc acatcttaac ggcgaaaaat cttttgtcaa acccgatttt taaattcccc        60

aactgtttgc cgtttctatc aacagtttgt tgcatttgca gacaatttgt tggcgaaaat       120

ctttgcagct ttgctgattc tccctcatta tttgaaatgt ggtttcactt tctgcaatta       180

aggtcggctt tgaatatctc ctctgcttta cgccaggttg ttcacggcac tcgctggcac       240

gctaaacgca agagctacaa agtgttgttc tggcgcgaga taaccccgct tgctgttcct       300

atcttcatgg agaatgcctg tgtcctgttg atgggggttc tgagcacttt tctggtcagc       360

tggctgggaa aagatgcgat ggccggcgtg ggattggcgg acagcttcaa tatggtcatt       420

atggcttttt ttgctgctat cgatcttggt actactgtcg ttgtggcatt tagtctcggt       480

aagcgggatc gacgacgagc gagggtggcg acgcggcagt cattggtgat catgacgttg       540

tttgccgtac tgttggcaac gcttattcat cattttggcg aacaaattat tgatttcgtc       600

gcgggtgatg ccacgacaga agttaaagca ctggcgttga cttatctgga gctgacggta       660

ctcagttatc cagcagctgc catcactctt attggtagcg gggcacttcg tggtgcaggg       720

aatacgaaaa taccgctatt gattaacggt agcctgaata ttcttaatat tattattagc       780

ggcatattga tttacggcct tttctcctgg ccgggactgg gatttgtcgg ggcagggctg       840

ggtttaacca tttctcgtta tattggcgca gttgcaattt tgtgggtgct ggcgattggt       900

tttaatcctg cgctaaggat ttcgttaaag agctatttta aaccgctgaa ttttagcatt       960

atctgggaag tcatggggat tggtattccc gcgagtgtcg aatcagtgtt atttaccagt      1020

ggtcggttat taacccaaat gttcgttgcc gggatgggga ccagtgttat tgccggaaat      1080

tttatcgcgt tttcaattgc ggctcttatc aacttacccg gaagtgcgct cggctctgct      1140

tctacgatca ttacaggccg aaggttgggg gtagggcaga tagcgcaagc agagattcag 1200

ttgcggcatg tgttctggct ttccactctt ggattaacgg ccatcgcctg gctaacggct 1260

ccctttgccg gggttatggc atcgttttac acccaggatc cacaggttaa acatgtcgtt 1320

gtgattctga tttggctaaa tgctttattt atgcctattt ggtccgcctc atgggtgcta 1380

cccgctggat ttaaaggtgc tcgtgatgcc cgttacgcca tgtgggtttc gatgttgagc 1440

atgtggggtt gtcgggttgt agtcggttat gtgctgggaa tcatgcttgg ctggggtgtg 1500

gttggtgtct ggatgggaat gtttgccgac tgggctgtgc gggccgtgct gttttactgg 1560

cgaatggtta ctggacgttg gctatggaaa taccctcgac ccgagccgca aaagtgtgaa 1620

aaaaagccag ttgtgtcgga ataa 1644

<210> 2
<211> 20
<212> DNA
<213> Artificial Sequence

<220>
<223> yeeO_HF1

<400> 2
cgtaactatg tctggcgctt 20

<210> 3
<211> 20
<212> DNA
<213> Artificial Sequence

<220>
<223> yeeO_HR1

<400> 3
aaacggcaaa cagttgggga 20

<210> 4
<211> 40
<212> DNA
<213> Artificial Sequence

<220>
<223> yeeO_PF

<400> 4
tccccaactg tttgccgttt gtgtaggctg gagctgcttc 40

<210> 5
<211> 40
<212> DNA
<213> Artificial Sequence

<220>
<223>    yeeO_PR

<400>    5
gtttattccg acacaactgg ctgtcaaaca tgagaattaa                              40

<210>    6
<211>    21
<212>    DNA
<213>    Artificial Sequence

<220>
<223>    yeeO_HF2

<400>    6
ccagttgtgt cggaataaac g                                                  21

<210>    7
<211>    20
<212>    DNA
<213>    Artificial Sequence

<220>
<223>    yeeO_HR2

<400>    7
agactcgatt aagcgcacga                                                    20

<210>    8
<211>    20
<212>    DNA
<213>    Artificial Sequence

<220>
<223>    yeeO_CF

<400>    8
cctatgttgc tcttgggctt                                                    20

<210>    9
<211>    20
<212>    DNA
<213>    Artificial Sequence

<220>
<223>    yeeO_CR

<400>    9
aggcaaagtg gcaattacgc                                                    20